(19) Europäisches Patentamt
European Patent Office
Office européen des brevets

(11) Veröffentlichungsnummer: **0 139 227**
**A2**

(12) # EUROPÄISCHE PATENTANMELDUNG

(21) Anmeldenummer: 84111146.1

(22) Anmeldetag: 19.09.84

(51) Int. Cl.[4]: A 01 N 43/50
A 01 N 43/56, A 01 N 43/653

(30) Priorität: 01.10.83 DE 3335767

(43) Veröffentlichungstag der Anmeldung:
02.05.85 Patentblatt 85/18

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Elbe, Hans-Ludwig, Dr.
Dasnöckel 59
D-5600 Wuppertal 11(DE)

(72) Erfinder: Jäger, Gerhard, Dr.
Gellertstrasse 18
D-5090 Leverkusen 1(DE)

(72) Erfinder: Krämer, Wolfgang, Dr.
Am Eckbusch 39/45
D-5600 Wuppertal 1(DE)

(72) Erfinder: Lunkenheimer, Winfried, Dr.
Bismarckstrasse 29
D-5600 Wuppertal 1(DE)

(72) Erfinder: Regel, Erik, Dipl.-Ing.
Untere Bergerheide 26
D-5600 Wuppertal 1(DE)

(72) Erfinder: Stetter, Jörg, Dr.
Gellertweg 4
D-5600 Wuppertal 1(DE)

(72) Erfinder: Fedtke, Carl, Dr.
Wehrheiderstrasse 5
D-5000 Köln-Dellbrück(DE)

(54) **Herbizide Mittel enthaltend Photosynthesehemmer-Herbizide in Kombination mit Azolylmethylketonen.**

(57) Die neuen synergistischen Wirkstoffkombinationen, bestehend aus (1) einem Photosynthesehemmer-Wirkstoff (Herbizid) und (2) einem Azolylmethylketon der allgemeinen Formel (II) (Synergist)

$$R - CO - CH_2 - Az \qquad (II)$$

in welcher
R für einen organischen Rest steht und
Az für einen Azolrest steht,

oder einem von deren Säureadditions-Salzen, weisen eine besonders hohe herbizide Wirksamkeit auf.

Charakteristische Beispiele für die Photosynthesehemmer-Wirkstoffe sind Metribuzin, Ametridione, Methabenzthiazuron, Linuron sowie 4-Amino-6-tert.-butyl-3-ethylthio-1,2,4-triazin-5-on.

EP 0 139 227 A2

BAYER AKTIENGESELLSCHAFT 5090 Leverkusen, Bayerwerk
Konzernverwaltung RP
Patentabteilung Bi/ABc
(III)

# Herbizide Mittel enthaltend Photosynthesehemmer-Herbizide in Kombination mit Azolylmethylketonen

Die vorliegende Erfindung betrifft neue herbizide synergistische Wirkstoffkombinationen, die aus bekannten Photosynthesehemmer-Herbiziden einerseits und aus bestimmten, weitgehend bekannten Azolylmethylketonen andererseits bestehen.

Es ist bereits bekannt geworden, daß bestimmte Herbizide, wie z.B. 4-Amino-6-tert.-butyl-3-methylthio- bzw. -ethylthio-1,2,4-triazin-5-on; 1-Amino-3-(2,2-dimethylpropyl)-6-(ethylthio)-1,3,5-triazin-2,4-dion; 1-Methoxy-1-methyl-3-(3,4-dichlorphenyl)-harnstoff oder 1,3-Dimethyl-1-(benzo-1,3-thiazol-2-yl)-harnstoff, photosynthese-hemmende Eigenschaften besitzen (vgl. z.B. Carl Fedtke, Biochemistry and Physiology of Herbicide Action, Springer Verlag, 1982). Nachteilig bei diesen heribziden Verbindungen ist jedoch, daß nicht immer alle vorkommenden Unkräuter und Ungräser voll erfaßt werden oder aber, daß bei entsprechend hohen Aufwandmengen einige Kulturpflanzenarten geschädigt werden.

Es wurde gefunden, daß die neuen Wirkstoffkombinationen, bestehend aus

a) einem Photosynthesehemmer-Wirkstoff (Herbizid) und

b) einem Azolylmethylketon der allgemeinen Formel (II) (Synergist),

$$R - CO - CH_2 - Az \qquad (II)$$

in welcher

R für einen organischen Rest steht und

Az für einen Azolrest steht,

oder einem von deren Säureadditions-Salzen, eine besonders hohe herbizide Wirksamkeit aufweisen.

Überraschenderweise ist die herbizide Wirksamkeit der erfindungsgemäßen Wirkstoffkombinationen wesentlich höher als die Summe der Wirkungen der einzelnen Wirkstoffe. Insbesondere besitzen die weitgehend bekannten Azolylmethylketone der allgemeinen Formel (II) bei den üblichen Aufwandmengen keine eigene herbizide Wirkung, bewirken aber eine Steigerung der herbiziden Wirkung der Photosynthesehemmer-Wirkstoffe. Somit ist der hier gefundene synergistische Effekt völlig unerwartet und überraschend.

Da der synergistische Effekt auch solche Unkräuter betrifft, die durch die verwendeten Photosynthesehemmer-Wirkstoffe bei alleiniger Ausbringung in üblichen Aufwandmengen nur unzureichend geschädigt oder gar nicht erfaßt werden, stellen die erfindungsgemäßen synergistischen Wirkstoffkombinationen eine wertvolle Bereicherung der Technik dar.

Als Photosynthesehemmer-Wirkstoffe für die erfindungsgemäßen Wirkstoffkombinationen seien vorzugsweise die folgenden der allgemeinen Formeln (I-A) bis (I-J) genannt:

(A) Triazinon-Derivate der Formel

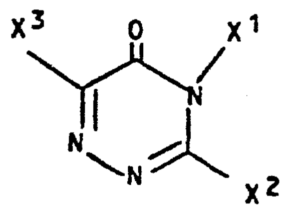

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

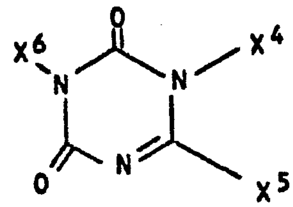

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

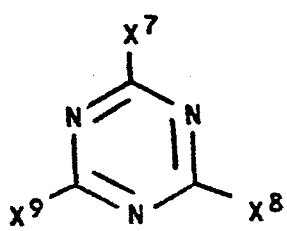

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

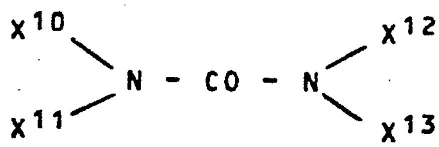

(I-D)

in welcher

$X^{10}$ für gegebenenfalls substituiertes Phenyl, Benzthiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad (I-E)$$

in welcher

$X^{14}$ für Alkyl mit bis zu 6 Kohlenstoffatomen, Alkoxy

mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

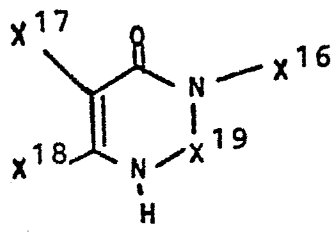

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO-oder $-SO_2$-Gruppe steht;

(G) Biscarbamat-Derivate der Formel

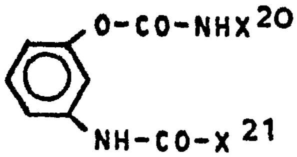

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

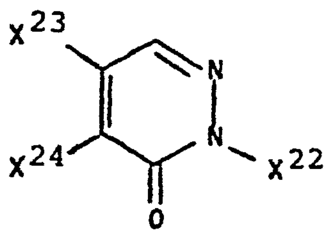

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

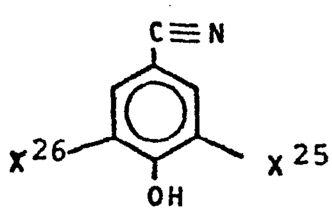

(I-J)

in welcher

$X^{25}$ für Halogen steht und

$X^{26}$ für Halogen steht.

Besonders bevorzugt sind folgende Photosynthesehemmer-Wirkstoffe der allgemeinen Formeln (I-A) bis (I-J):

(A) Triazinon-Derivate der Formeln

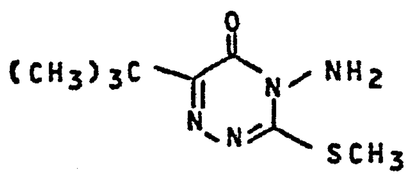

(I-A-1)
(Metribuzin)

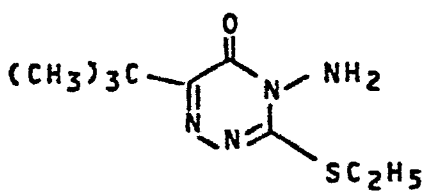

(I-A-2)

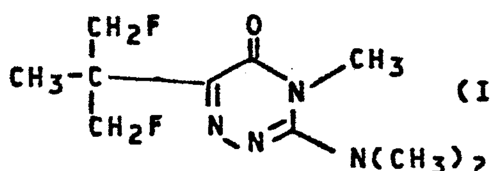

(I-A-3)

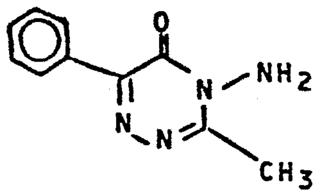

(I-A-4)
(Metamitron)

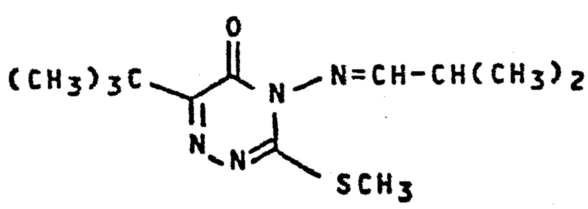

(I-A-5)
(Isomethiozin)

(B) Triazindion-Derivate der Formeln

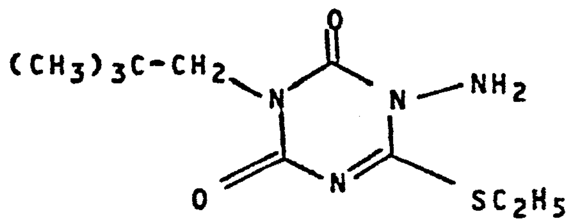

(I-B-1)
(Ametridione)

(C) Triazin-Derivate der Formeln

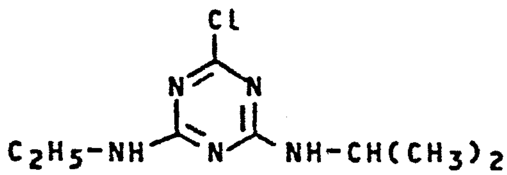

(I-C-1)
(Atrazine)

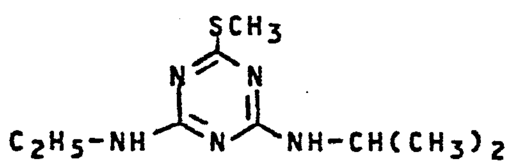

(I-C-2)
(Ametryne)

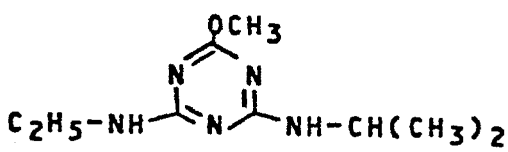

(I-C-3)
(Atraton)

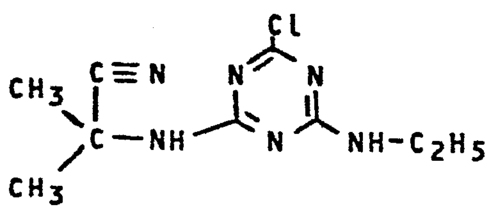

(I-C-4)
(Cyanazine)

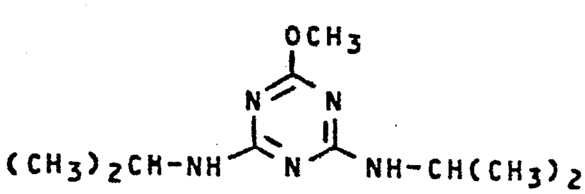

(I-C-5)
(Prometon)

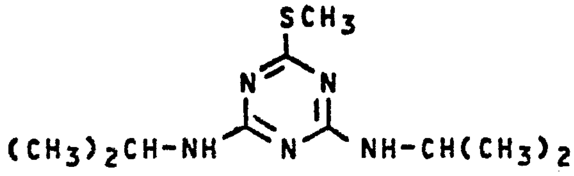

(I-C-6)
(Prometryne)

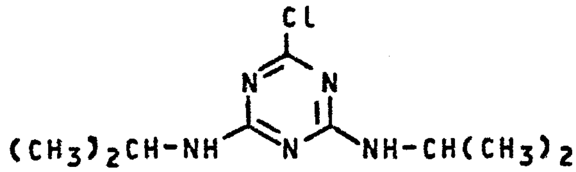

(I-C-7)
(Propazine)

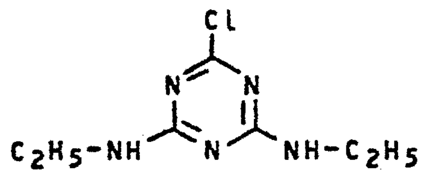

(I-C-8)
(Simazine)

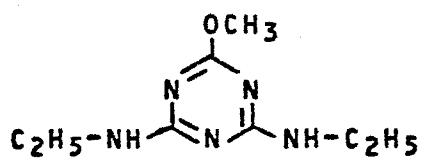

(I-C-9)
(Simeton)

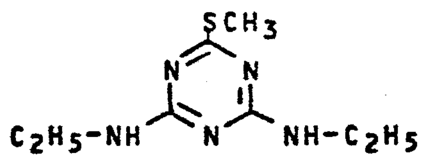

(I-C-10)
(Simetryne)

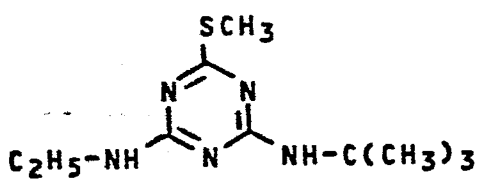

(I-C-11)
(Terbutryne)

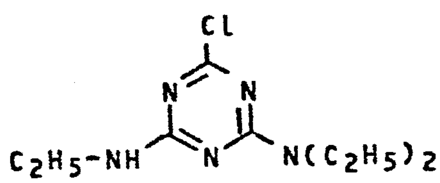

(I-C-12)
(Trietazine)

(D) Harnstoff-Derivate der Formeln

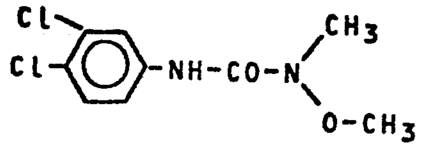

(I-D-1)
(Linuron)

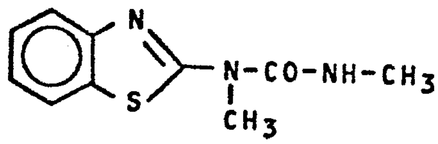

(I-D-2)
(Methabenzthiazuron)

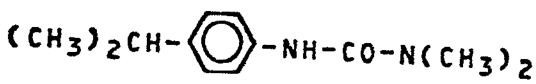

(I-D-3)
(Isoproturon)

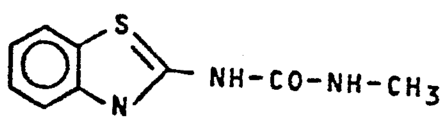

(I-D-4)
(Benzthiazuron)

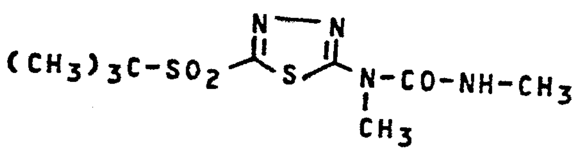

(I-D-5)
(Buthiuron)

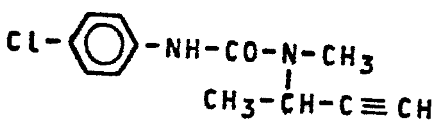

(I-D-6)
(Buturon)

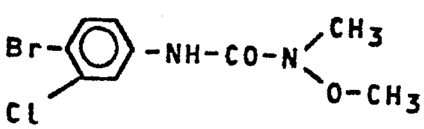

(I-D-7)
(Chlorbromuron)

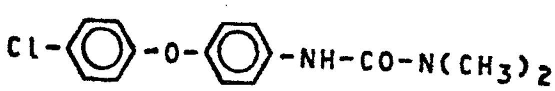

(I-D-8)
(Chloroxuron)

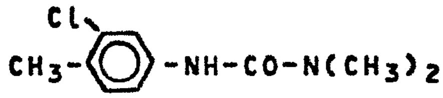

(I-D-9)
(Chlortoluron)

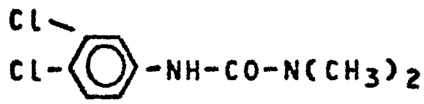

(I-D-10)
(Diuron)

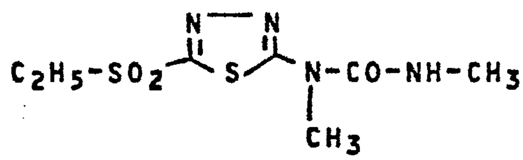

(I-D-11)
(Ethidimuron)

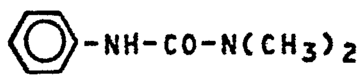

(I-D-12)
(Fenuron)

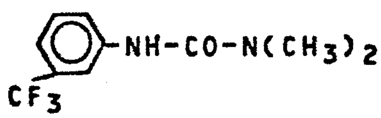

(I-D-13)
(Fluometuron)

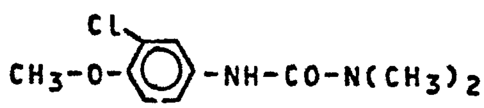

(I-D-14)
(Metoxuron)

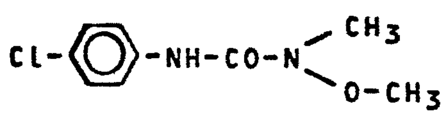

(I-D-15)
(Monolinuron)

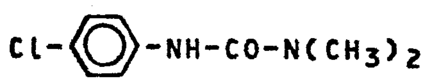

(I-D-16)
(Monuron)

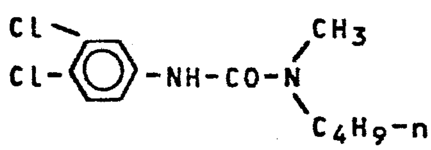

(I-D-17)
(Neburon)

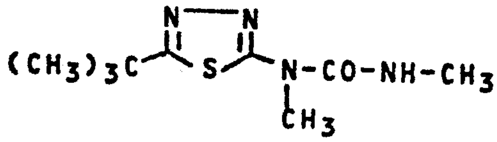

(I-D-18)
(Tebuthiuron)

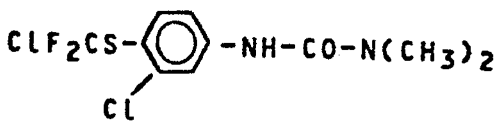

(I-D-19)
(Thiochlormethyl)

(E) Carboxanilid-Derivate der Formeln

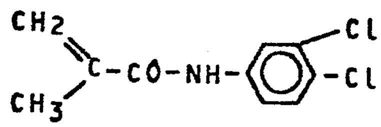

(I-E-1)
(Chlorancryl)

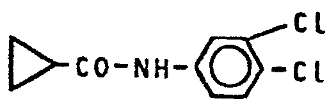

(I-E-2)
(Cypromid)

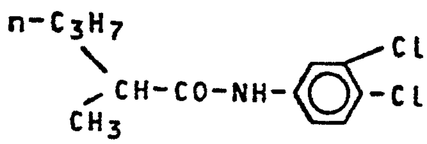

(I-E-3)
(Karsil)

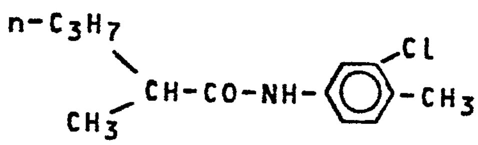

(I-E-4)
(Pentanochlor)

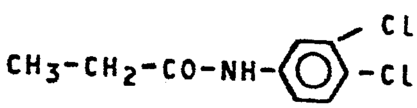

(I-E-5)
(Propanil)

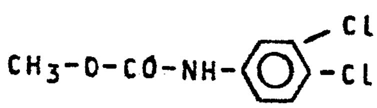

(I-E-6)
(Swep)

(F) Uracil-Derivate der Formeln

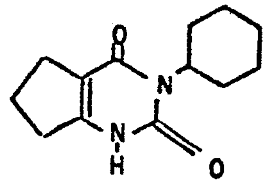

(I-F-1)
(Lenacil)

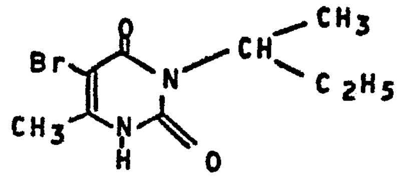

(I-F-2)
(Bromacil)

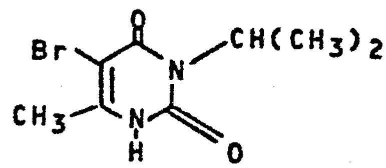

(I-F-3)
(Isocil)

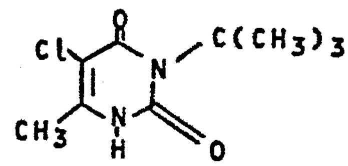

(I-F-4)
(Terbacil)

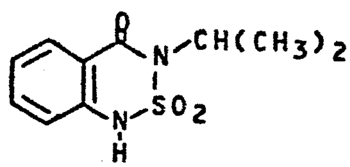

(I-F-5)
(Bentazon)

(G) Biscarbamat-Derivate der Formeln

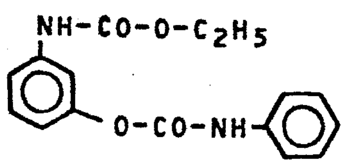

(I-G-1)
(Desmedipham)

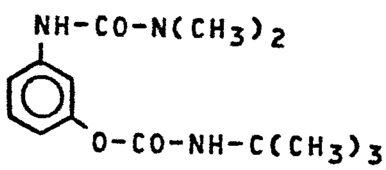

(I-G-2)
(Karbutilate)

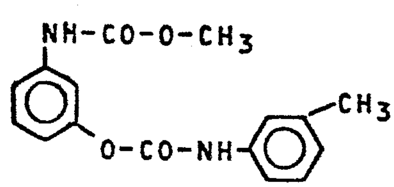

(I-G-3)
(Phenmedipham)

(H) Pyridazinon-Derivate der Formeln

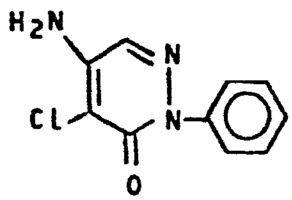

(I-H-1)
(Pyrazone)

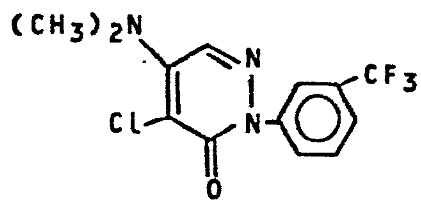

(I-H-2)
(Metflurazone)

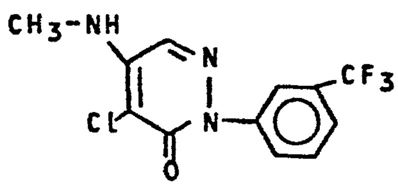

(I-H-3)
(Norflurazon)

(J) Hydroxybenzonitril-Derivate der Formeln

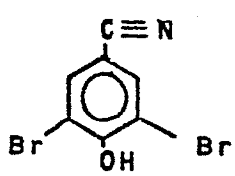

(I -J-1)
(Bromoxynil)

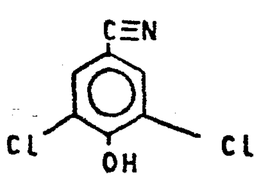

(I -J-2)
(Chloroxynil)

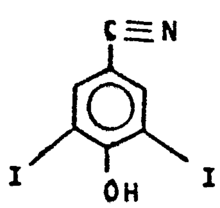

(I -J-3)
(Ioxynil)

Die Photosynthesehemmer-Wirkstoffe der Formeln (I-A) bis (I-J) sind bekannt (vgl. z.B. Carl Fedtke, Biochemistry and Physiology of Herbicide Action, Springer-Verlag, 1982).

Die weiterhin als Mischungskomponente zu verwendenden Azolylmethylketone sind durch die Formel (II) allgemein definiert. In dieser Formel stehen vorzugsweise

R für gegebenenfalls substituiertes Alkyl sowie für einen gegebenenfalls substituierten cycloaliphatischen Rest, der bis zu 2 Sauerstoffatome als Heteroatome enthalten kann; und

Az für Triazolyl, Imidazol oder Pyrazolyl.

Bevorzugt sind Verbindungen der Formel (II), in denen

R für gegebenenfalls substituiertes Cycloalkyl, für gegebenenfalls substituiertes Dioxolanyl, für gegebenenfalls substituiertes Dioxanyl oder für die Gruppierungen

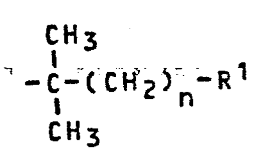 , 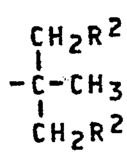 und 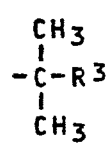 steht, wobei

0139227

$R^1$ für Wasserstoff, Halogen, Cyano, Alkyl, Alkenyl, Alkinyl, Alkoxycarbonyl, gegebenenfalls substituiertes Aryl oder die Gruppierung $-X-R^4$ steht;

$R^2$ für Halogen steht;

$R^3$ für die CHO-Gruppe und deren Derivate, wie Oxime, Oximether, kettenförmige Acetale oder ringförmige Acetale (gegebenenfalls substituierte Dioxolane und Dioxane) steht;

$R^4$ für Alkyl, Halogenalkyl, Cyano, gegebenenfalls substituiertes Aryl oder gegebenenfalls substituiertes Aralkyl steht;

X für Sauerstoff oder Schwefel steht; der Index

n für die Zahlen 0, 1 oder 2 steht; und

Az für 1,2,4-Triazol-1-yl, 1,2,4-Triazol-4-yl, Imidazol-1-yl oder Pyrazol-1-yl steht.

Besonders bevorzugt sind Verbindungen der Formel (II) in denen

R für gegebenenfalls einfach bis dreifach, gleich oder verschieden substituiertes Cycloalkyl mit 3 bis 7 Kohlenstoffatomen steht, wobei als Substituenten genannt seien: Alkyl mit 1 bis 4 Kohlenstoffatomen, Halogen und gegebenenfalls substituiertes Phenyl; weiterhin für jeweils gegebenenfalls einfach bis

vierfach, gleich oder verschieden substituiertes Dioxolan-2-yl oder 1,3-Dioxanyl steht, wobei als Substituenten genannt seien: Alkyl mit 1 bis 4 Kohlenstoffatomen sowie jeweils gegebenenfalls einfach bis fünffach, gleich oder verschieden substituiertes Phenyl und Phenoxyalkyl mit 1 bis 4 Kohlenstoffatomen im Alkylteil, wobei jeweils als Phenylsubstituenten genannt seien; Halogen, Alkyl mit 1 bis 4 Kohlenstoffatomen, Alkoxy und Alkylthio mit jeweils 1 bis 2 Kohlenstoffatomen sowie Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratomen; sowie für die Gruppierungen

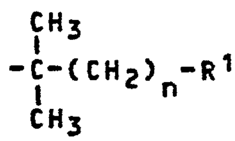 , 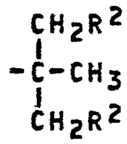 und 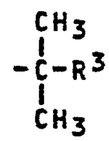 steht, wobei

$R^1$ für Wasserstoff, Halogen, Cyano, geradkettiges oder verzweigtes Alkyl mit 1 bis 4 Kohlenstoffatomen, Alkenyl und Alkinyl mit jeweils 2 bis 6 Kohlenstoffatomen, Alkoxycarbonyl mit 1 bis 4 Kohlenstoffatomen im Alkylteil und für gegebenenfalls einfach bis fünffach, gleich oder verschieden substituiertes Phenyl steht, wobei als Substituenten genannt seien: Halogen, Alkyl mit 1 bis 4 Kohlenstoffatomen, Halogenalkyl, Halogenalkoxy und Halogenalkylthio mit jeweils 1 bis 2 Kohlenstoffatomen und 1 bis 5 gleichen oder verschiedenen Halogenatomen, wie Fluor- und Chloratomen, Alkoxycarbonyl mit 1 bis 4 Kohlenstoffatomen im Alkylteil, Alkylcarbonyl mit 1 bis 4 Kohlenstoffatomen im Alkylteil, Cyano, Nitro sowie jeweils gegebenenfalls durch Halogen substituiertes Phenyl und Phenoxy; sowie für die Gruppierung $-X-R^4$ steht;

$R^2$ für Fluor oder Chlor steht;

$R^3$ für die CHO-Gruppe, Alkoximinomethyl mit 1 bis 4 Kohlenstoffatomen im Alkylteil, Dialkoxymethyl mit 1 bis 4 Kohlenstoffatomen in jedem Alkylteil sowie für jeweils gegebenenfalls einfach bis vierfach, gleich oder verschieden substituiertes Dioxolan-2-yl oder 1,3-Dioxanyl steht, wobei als Substituenten die bei R für diese Reste bereits genannten Substituenten infrage kommen;

$R^4$ für geradkettiges oder verzweigtes Alkyl mit 1 bis 4 Kohlenstoffatomen, Halogenalkyl mit 1 bis 2 Kohlenstoffatomen und 1 bis 5 gleichen oder verschiedenen Halogenatomen, wie Fluor- und Chloratomen, Cyano sowie für jeweils gegebenenfalls einfach bis fünffach, gleich oder verschieden substituiertes Phenyl und Phenylalkyl mit 1 bis 4 Kohlenstoffatomen im Alkylteil steht, wobei jeweils als Phenylsubstituenten die bei $R^1$ bereits genannten Phenylsubstituenten infrage kommen;

X der Index n und Az für die oben angegebenen Bedeutungen stehen.

Insbesondere bevorzugt sind Verbindungen der Formel (II) in denen

R für gegebenenfalls einfach bis dreifach, gleich oder verschieden substituiertes Cycloalkyl mit 3 bis 6 Kohlenstoffatomen steht, wobei als Substituenten genannt seien: Methyl, Ethyl, Isopropyl, tert.-Butyl, Chlor, Brom sowie gegebenenfalls durch Chlor oder Fluor substituiertes Phenyl; weiterhin für jeweils gegebenenfalls einfach bis dreifach, gleich oder verschieden substituiertes Dioxolan-2-yl, 1,3-Dioxan-5-yl oder 1,3-Dioxan-2-yl steht, wobei als Substituenten genannt seien: Methyl, Ethyl, n-Propyl, Isopropyl sowie jeweils gegebenenfalls einfach bis dreifach, gleich oder verschieden durch Fluor, Chlor, Methyl, Trifluormethyl oder Trifluormethoxy substituiertes Phenyl oder Phenoxymethyl; sowie für die Gruppierungen

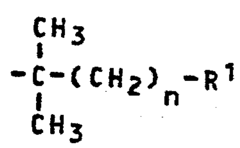 , 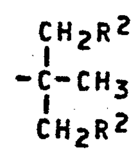 und 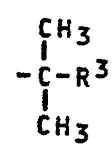 steht, wobei

$R^1$ für Wasserstoff, Fluor, Chlor, Brom, Cyano, Methyl, Ethyl, Propyl, tert.-Butyl, Vinyl, Propargyl, Methoxycarbonyl, Ethoxycarbonyl sowie gegebenenfalls einfach bis dreifach, gleich oder verschieden substituiertes Phenyl steht, wobei als Substituenten genannt seien: Fluor, Chlor, Brom, Methyl, Ethyl, Isopropyl,

tert.-Butyl, Methoxy, Methylthio, Trifluormethyl, Trifluormethoxy, Trifluormethylthio, Methoxycarbonyl, Ethoxycarbonyl, Methylcarbonyl, Ethylcarbonyl, Cyano, Nitro, gegebenenfalls durch Chlor substituiertes Phenyl, sowie gegebenenfalls durch Chlor substituiertes Phenoxy; sowie für die Gruppierung $-X-R^4$ steht;

$R^2$ für Fluor oder Chlor steht;

$R^3$ für die CHO-Gruppe, Methoximinomethyl, Ethoximinomethyl, Dimethoxymethyl sowie für jeweils gegebenenfalls einfach bis dreifach, gleich oder verschieden subtituiertes Dioxolan-2-yl, 1,3-Dioxan-5-yl oder 1,3-Dioxan-2-yl steht, wobei als Substituenten die bei R für diese Reste bereits genannten Substituenten infrage kommen;

$R^4$ für Methyl, Ethyl, Trifluormethyl, Cyano, sowie für jeweils gegebenenfalls einfach bis dreifach, gleich oder verschieden substituiertes Phenyl und Benzyl steht, wobei jeweils als Phenylsubstituenten die bei $R^1$ bereits genannten Phenylsubstituenten infrage kommen;

X der Index n und Az für die oben genannten Bedeutungen stehen.

Bevorzugte Verbindungen sind auch Additionsprodukte aus Säuren und denjenigen Azolylmethylketonen der Formel (II), in denen die Substituenten R und Az die Bedeutungen haben, die bereits vorzugsweise für diese Substituenten genannt

wurden.

Zu den Säuren, die addiert werden können, gehören vorzugsweise Halogenwasserstoffsäure, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Die Azolylmethylketone der Formel (II) sind bereits in der Literatur beschrieben (vergleiche hierzu z.B. DE-OS 24 31 407 [Le A 15 735], EP 0 031 911 [Le A 20 067], EP 0 044 993 [Le A 20 458], DE-OS 30 10 560, EP 0 054 865 [Le A 20 763], EP 0 080 096 [Le A 21 400], EP 0 081 675 [Le A 21 383], EP 0 043 923 sowie die deutschen Patentanmeldungen P 32 09 431 vom 1.6.3.1982 [Le A 21 585], P 32 22 220 vom 12.6.1982 [Le A 21 712], P 32 24 129 vom 29.6.1982, P 32 29 274 vom 5.8. 1982 [Le A 21 844] und P 3242 222 vom 15.11.1982 [Le A 21 980]); bzw. können sie nach im Prinzip bekannten Verfahren hergestellt werden, wie durch Umsetzung der entsprechenden Halogenmethylketone mit Azol in Gegenwart eines inerten organischen Lösungsmittels, wie beispielsweise Aceton oder Acetonitril, und in Gegenwart eines Säurebindemittels, wie beispielsweise Kaliumcarbonat oder Triethylamin, bei Temperaturen zwischen 20 und 150°C.

Die Säureadditions-Salze der Verbindungen der Formel (II) können anschließend nach üblichen Salzbildungsmethoden, z.B. durch Lösen einer Verbindung der Formel (II) in einem geeigneten inerten Lösungsmittel und Hinzufügen der Säure, erhalten werden.

Die Gewichtsverhältnisse der Wirkstoffe in den neuen Wirkstoffkombinationen können in relativ großen Bereichen schwanken. Im allgemeinen entfallen auf ein Gewichtsteil Photosynthesehemmer-Wirkstoff (herbizider Wirkstoff) 0,25 bis 100, vorzugsweise 5 bis 50, insbesondere 10 bis 20, Gew.-Teile Azolylmethylketon der Formel (II) (Synergist).

Die Photosynthesehemmer-Wirkstoffe weisen starke herbizide Wirkungen auf. Dennoch besitzen sie gegen einige Unkräuter, wie z.B. Galium aparine, Ipomoea hederacea, Datura stramonium, Cirsium arrense, Convolvulus arvensis oder Solanum nigrum und einige Ungräser, wie z.B. Agropyron repens, Avena fatua, Cynodon dactylon, Cyperus ssp. und Colium rigidum eine nicht immer ausreichende Wirkung. Die erfindungsgemäßen Wirkstoffkombinationen dehnen das Wirkungsspektrum der Verbindungen der Formeln (I-A bis I-J) aus und ermöglichen dadurch eine Bekämpfung dieser durch die herbiziden Wirkstoffe alleine nur schwer oder gar nicht bekämpfbaren Unkräuter.

Die erfindungsgemäßen Wirkstoffkombinationen können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffkombinationen ist jedoch keineswegs auf diese Gattungen beschränkt, sondern erstreckt sich in gleicher Weise auch auf andere Pflanzen.

Die erfindungsgemäßen Wirkstoffkombinationen zeigen insbesondere neben einer guten Wirkung gegen grasartige Unkräuter auch eine gute herbizide Wirkung bei breitblättrigen Unkräutern.

Die erfindungsgemäßen Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

In den Formulierungen können als weitere Zusätze Farbstoffe wie anorganische Pigmente, z.B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffkombination, vorzugsweise zwischen 0,5 und 90 %.

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch als Einzelformulierungen bei der Anwendung gemischt, d.h. in Form von Tankmischungen zur Anwendung gebracht werden.

Die neuen Wirkstoffkombinationen können als solche oder in ihren Formulierungen weiterhin auch in Mischung mit anderen bekannten Herbiziden Verwendung finden, wobei wiederum Fertigformulierungen oder Tankmischungen möglich sind. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die erfindungsgemäßen Wirkstoffkombinationen können sowohl vor als auch nach der Einsaat ebenso wie nach dem Auflaufen der Pflanzen gemeinsam oder in getrennten Anwendungen ausgebracht werden. Hierbei spielt die Reihenfolge der Anwendungen keine Rolle.

Beim Einsatz der erfindungsgemäßen Synergisten kann die übliche Aufwandmenge der Herbizide der Formeln (I-A bis I-J) verringert werden. Die Aufwandmenge an herbizidem Photosynthesehemmer-Wirkstoff liegt bei Flächenbehandlung zwischen 0,01 und 3,0 kg/ha, vorzugsweise zwischen 0,05 und 2,0 kg/ha.

Die Aufwandmenge an synergistischem Azolylmethylketon (II) liegt bei Flächenbehandlung zwischen 0,1 und 10 kg/ha, vorzugsweise zwischen 0,5 und 3 kg/ha.

Die gute herbizide Wirkung der erfindungsgemäßen Wirkstoffkombinationen geht aus den nachfolgenden Beispielen hervor. Während die einzelnen Wirkstoffe in der herbiziden Wirkung Schwächen aufweisen, zeigen die Kombinationen eine Unkrautwirkung, die über eine einfache Wirkungssummierung hinausgeht.

Ein synergistischer Effekt liegt bei Herbiziden immer dann vor, wenn die herbizide Wirkung der Wirkstoffkombination größer ist als die Summe der Wirkungen der applizierten Wirkstoffe.

## Beispiel A

Pre-emergence-Test

Lösungsmittel: 5 Gew.-Teile Aceton
Emulgator: 1 Gew.-Teil Alkylarylpolyglykolether

Zur Herstellung einer zweckmäßigen Wirkstoffzubereitung vermischt man 1 Gew.-Teil herbiziden Wirkstoffs bzw. Synergisten bzw. eines Gemisches aus herbizidem Wirkstoff und Synergisten mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit einer Herbizid-Zubereitung bzw. mit der Synergisten-Zubereitung bzw. mit der Zubereitung aus Synergisten und herbizidem Wirkstoff begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Wirkstoffe, Aufwandmenge und Resultate gehen aus den nachfolgenden Tabellen hervor.

**Tabelle $A_1$: pre-emergence-Test**

Synergistische Wirkung von Azolylmethylketonen (II) (=Synergist S) und 4-Amino-6-tert.-butyl-3-methylthio-1,2,4-triazin-5-on (I-A-1) (= Herbizid H) an Ipomoea hederacea. Die Aufwandmenge in kg/ha bezieht sich auf den Gehalt an Wirkstoff.

| Struktur des Synergisten(II) | (S) kg/ha | (H) kg/ha | % Wirkung bei Ipomoea hederacea H | S | H+S |
|---|---|---|---|---|---|
| 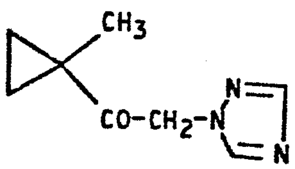 | 0,5 | 0,05 | 0 | 0 | 20 |
| | 2,0 | 0,05 | 0 | 0 | 50 |
| | 0,5 | 0,15 | 20 | 0 | 100 |
| (II-1) | 2,0 | 0,15 | 20 | 0 | 100 |
| 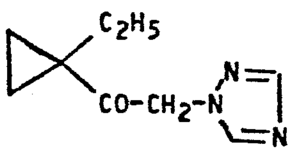 | 0,5 | 0,05 | 0 | 0 | 30 |
| | 2,0 | 0,05 | 0 | 0 | 50 |
| | 0,5 | 0,15 | 10 | 0 | 90 |
| (II-2) | 2,0 | 0,15 | 10 | 0 | 100 |
| 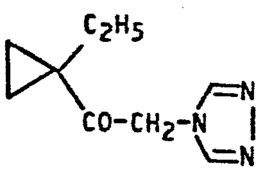 | 0,5 | 0,05 | 0 | 0 | 0 |
| | 2,0 | 0,05 | 0 | 0 | 0 |
| | 0,5 | 0,15 | 10 | 0 | 50 |
| (II-3) | 2,0 | 0,15 | 10 | 0 | 50 |
| 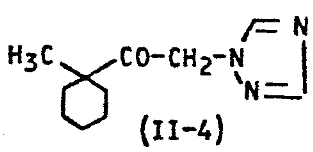 | 0,5 | 0,05 | 0 | 0 | 20 |
| | 2,0 | 0,05 | 0 | 0 | 30 |
| | 0,5 | 0,15 | 20 | 0 | 40 |
| (II-4) | 2,0 | 0,15 | 20 | 0 | 90 |
| 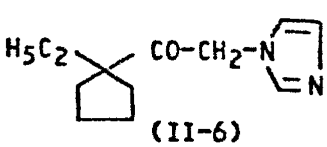 | 0,5 | 0,05 | 0 | 0 | 0 |
| | 2,0 | 0,05 | 0 | 0 | 50 |
| | 0,5 | 0,15 | 20 | 0 | 80 |
| (II-6) | 2,0 | 0,15 | 20 | 0 | 60 |

Tabelle A1: (Fortsetzung)

| Struktur des Synergisten (II) | (S) Kg/ha | (H) kg/ha | % Wirkung bei Ipomoea hederacea H | S | H+S |
|---|---|---|---|---|---|
| 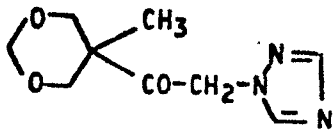 (II-7) | 0,5 | 0,05 | 10 | 0 | 30 |
| | 2,0 | 0,05 | 10 | 0 | 100 |
| | 0,5 | 0,15 | 30 | 0 | 100 |
| | 2,0 | 0,15 | 30 | 0 | 100 |
| 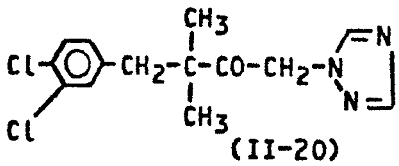 (II-20) | 0,5 | 0,05 | 10 | 0 | 0 |
| | 2,0 | 0,05 | 10 | 0 | 0 |
| | 0,5 | 0,15 | 20 | 0 | 80 |
| | 2,0 | 0,15 | 20 | 0 | 50 |
| 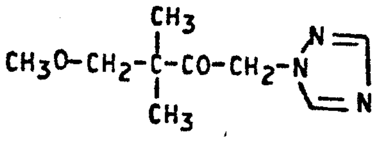 (II-30) | 0,5 | 0,05 | 10 | 0 | 60 |
| | 2,0 | 0,05 | 10 | 0 | 80 |
| | 0,5 | 0,15 | 20 | 0 | 100 |
| | 2,0 | 0,15 | 20 | 0 | 100 |
| 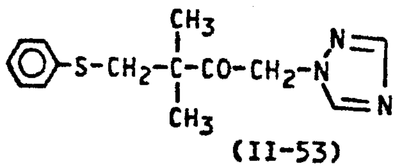 (II-53) | 0,5 | 0,05 | 0 | 0 | 10 |
| | 2,0 | 0,05 | 0 | 0 | 10 |
| | 0,5 | 0,15 | 10 | 0 | 20 |
| | 2,0 | 0,15 | 10 | 0 | 40 |
| 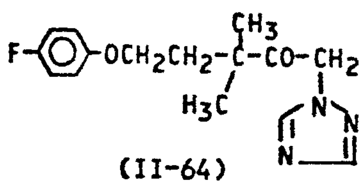 (II-64) | 0,5 | 0,05 | 0 | 0 | 50 |
| | 2,0 | 0,05 | 0 | 0 | 100 |
| | 0,5 | 0,15 | 10 | 0 | 70 |
| | 2,0 | 0,15 | 10 | 0 | 100 |
| 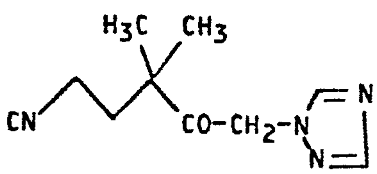 x HCl (II-89) | 0,5 | 0,05 | 0 | 0 | 0 |
| | 2,0 | 0,05 | 0 | 0 | 20 |
| | 0,5 | 0,15 | 0 | 0 | 40 |
| | 2,0 | 0,15 | 0 | 0 | 50 |

Tabelle $A_1$: (Fortsetzung)

Pre-emergence-Test

| Struktur des Synergisten (II) | (S) kg/ha | (H) kg/ha | % Wirkung bei Ipomoea hederacea H | S | H+S |
|---|---|---|---|---|---|
| 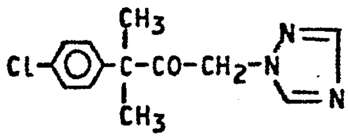 | 0,5 | 0,05 | 0 | 0 | 10 |
| | 2,0 | 0,05 | 0 | 0 | 10 |
| | 0,5 | 0,15 | 10 | 0 | 50 |
| (II-90) | 2,0 | 0,15 | 10 | 0 | 50 |
| 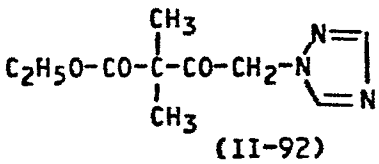 | 0,5 | 0,05 | 0 | 0 | 0 |
| | 2,0 | 0,05 | 0 | 0 | 20 |
| | 0,5 | 0,15 | 10 | 0 | 40 |
| (II-92) | 2,0 | 0,15 | 10 | 0 | 40 |
| 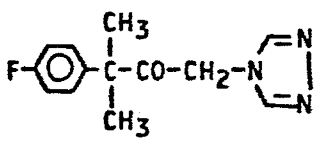 | 0,5 | 0,05 | 0 | 0 | 10 |
| | 2,0 | 0,05 | 0 | 0 | 20 |
| | 0,5 | 0,15 | 10 | 0 | 30 |
| (II-94) | 2,0 | 0,15 | 10 | 0 | 50 |
| 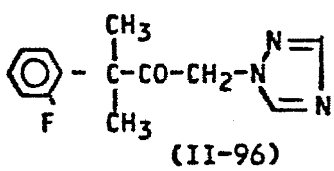 | 0,5 | 0,05 | 0 | 0 | 0 |
| | 2,0 | 0,05 | 0 | 0 | 10 |
| | 0,5 | 0,15 | 0 | 0 | 70 |
| (II-96) | 2,0 | 0,15 | 0 | 0 | 70 |

| Struktur des Synergisten(II) | (S) kg/ha | (H) kg/ha | % Schäden an Ipomoea hederacea H | S | H+S |
|---|---|---|---|---|---|
| 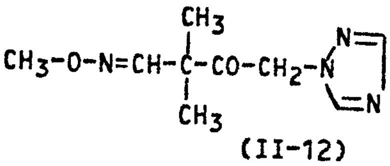 (II-12) | 0,03 | 0,1 | 10 | | 10 |
| | 0,1 | 0,1 | 10 | | 10 |
| | 0,3 | 0,1 | 10 | | 10 |
| | 1 | 0,1 | 10 | | 10 |
| | 2 | 0,1 | 10 | 0 | 90 |
| 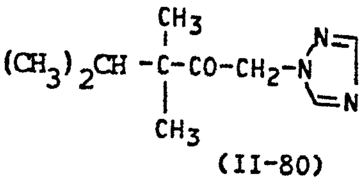 (II-80) | 0,03 | 0,1 | 0 | | 40 |
| | 0,1 | 0,1 | 0 | | 60 |
| | 0,3 | 0,1 | 0 | | 100 |
| | 1 | 0,1 | 0 | | 100 |
| | 2 | 0,1 | 0 | 0 | 100 |
| 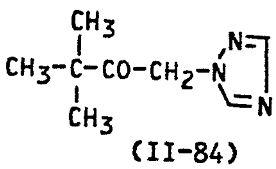 (II-84) | 0,03 | 0,1 | 0 | | 30 |
| | 0,1 | 0,1 | 0 | | 70 |
| | 0,3 | 0,1 | 0 | | 80 |
| | 1 | 0,1 | 0 | | 90 |
| | 2 | 0,1 | 0 | 0 | 90 |
| 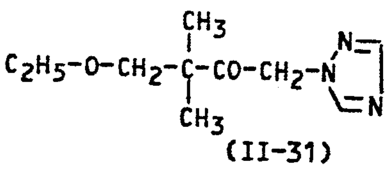 (II-31) | 0,03 | 0,1 | 10 | | 0 |
| | 0,1 | 0,1 | 10 | | 0 |
| | 0,3 | 0,1 | 10 | | 10 |
| | 1 | 0,1 | 10 | | 50 |
| | 2 | 0,1 | 10 | 10 | 80 |
| 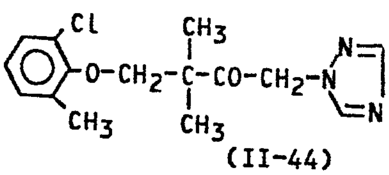 (II-44) | 0,03 | 0,1 | 10 | | 10 |
| | 0,1 | 0,1 | 10 | | 10 |
| | 0,3 | 0,1 | 10 | | 30 |
| | 1 | 0,1 | 10 | | 100 |
| | 2 | 0,1 | 10 | 0 | 100 |
| 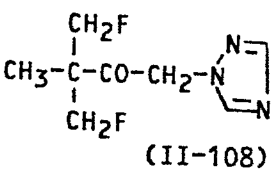 (II-108) | 0,03 | 0,1 | 10 | | 30 |
| | 0,1 | 0,1 | 10 | | 30 |
| | 0,3 | 0,1 | 10 | | 40 |
| | 1 | 0,1 | 10 | | 50 |
| | 2 | 0,1 | 10 | 0 | 80 |

Tabelle $A_1$:(Fortsetzung)

| Struktur des Synergisten (II) | (S) kg/ha | (H) kg/ha | % Schäden an Ipomoea hederacea H | S | H+S |
|---|---|---|---|---|---|
| 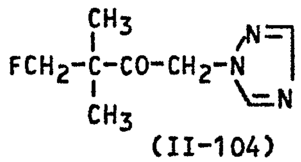 | 0,03 | 0,1 | 10 | | 20 |
| | 0,1 | 0,1 | 10 | | 80 |
| | 0,3 | 0,1 | 10 | | 90 |
| | 1 | 0,1 | 10 | | 90 |
| (II-104) | 2 | 0,1 | 10 | 0 | 90 |

Die alleinige Anwendung des Herbizids (I-A-1) in einer Konzentration von 0,5 bzw. 2,0 kg/ha führt bei Ipomoea hederacea zu einer Schädigung von 80 bzw. 95 %.

Tabelle $A_2$

Synergistische Wirkung des Azolylmethylketons gemäß Herstellungsbeispiel (II-6) und verschiedenen Photosynthesehemmer-Herbiziden an Ipomoea hederacea. Die Aufwandmenge in kg/ha bezieht sich auf den Gehalt an Wirkstoff.

| Synergist | Herst. Bsp. (II-6) | 0 kg/ha | 0,5 kg/ha | 2,0 kg/ha |
|---|---|---|---|---|
| Herbizid | kg/ha | % Wirkung bei Ipomoea hederacea | | |
| Metribuzin (I-A-1) | 0,07 | 10 | 30 | 50 |
| | 0,1 | 10 | 30 | 80 |
| Ametridione (I-B-1) | 0,5 | 30 | 50 | 100 |
| | 1,0 | 40 | 100 | 100 |
| (I-A-2) | 1,0 | 20 | 80 | 90 |
| Methabenzthiazuron (I-D-2) | 3,0 | 50 | 80 | 100 |
| Linuron (I-D-1) | 0,5 | 20 | 10 | 80 |
| - | 0 | 0 | 0 | 0 |

Herstellungsbeispiele

Beispiel 1

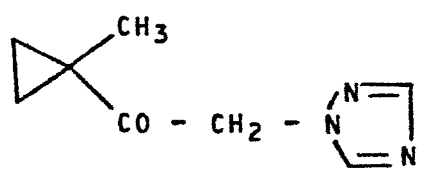

140 ml Triethylamin werden in ein Gemisch von 69 g (1 Mol) Triazol, 185,8 g (1 Mol) Brommethyl-(1-methylcyclopropyl)-keton und 250 ml Acetonitril getropft.
Das Gemisch wird bei Raumtemperatur 12 Stunden gerührt. Das ausgeschiedene Triethylammoniumbromid wird abgesaugt und das Filtrat im Vakuum eingedampft. Der ölige Rückstand wird mit 50 ml Wasser verrührt und mit Chloroform extrahiert. Man erhält 151,5 g (91,5 % der Theorie) 1-Methylcyclopropyl-(1,2,4-triazol-1-yl-methyl)-keton vom Schmelzpunkt 55-58°C.

Herstellung des Ausgangsproduktes

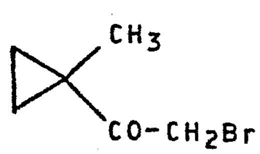

Eine Lösung von 25,6 g (0,5 Mol) Brom in 125 ml Chloroform wird in eine Lösung von 4,9 g (0,5 Mol) Methyl-(1-methylcyclopropyl)-keton in 250 ml Methanol getropft. Nach Entfärbung wird die Lösung auf 1000 ml Eiswasser gegeben. Die Chloroformphase wird abgetrennt, mit Wasser neutral gewaschen, über Natriunsulfat getrocknet und eingedampft. Man erhält 90,2 g Brommethyl-(1-methylcyclopropyl)-keton vom Brechungsindex $n_D^{20}$= 1,5045.

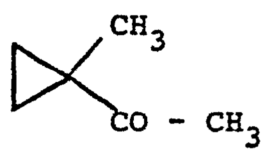

793 g (5,9 Mol) 5-Chlor-3-methylpentan-2-on werden bei 70°C in eine Lösung von 390 g (7,1 Mol) Kaliumhydroxid in 350 ml Wasser getropft. Das Gemisch wird 4 Stunden auf 80°C erhitzt, dann auf Raumtemperatur abgekühlt und mit verdünnter Schwefelsäure neutralisiert. Die organische Phase wird abgetrennt, über Natriumsulfat getrocknet und destilliert. Man erhält 462,4 g (80 % der Theorie) Methyl-(1-methylcyclopropyl)-keton vom Siedepunkt 126-158°C.

In analoger Weise können die folgenden Verbindungen der allgemeinen Formel

$$R - CO - CH_2 - Az \qquad (II)$$

erhalten werden.

Tabelle B

| Bsp. Nr | R | Az | Fp(°C) bzw. Kp(°C)/mbar bzw. $n_D^{20}$ bzw. $R_f$ |
|---|---|---|---|
| II-2 | 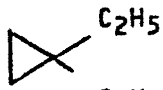 | 1,2,4-Triazol-1-yl | 64 |
| II-3 | 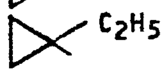 | 1,2,4-Triazol-4-yl | 148 |
| II-4 | 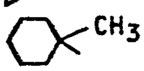 | 1,2,4-Triazol-1-yl | 58-60 |
| II-5 | 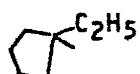 | 1,2,4-Triazol-1-yl | 1,5027 |
| II-6 | 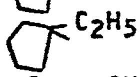 | Imidazol-1-yl | 75-76 |
| II-7 | 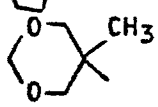 | 1,2,4-Triazol-1-yl | 99 |
| II-8 | 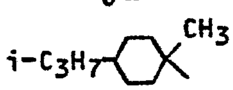 | 1,2,4-Triazol-1-yl | 99-101 |

**Tabelle B (Fortsetzung)**

| Bsp. Nr. | R | Az | Fp(°C) bzw. Kp(°C) /mbar bzw. $n_D^{20}$ bzw. $R_f$ |
|---|---|---|---|
| II-9 | 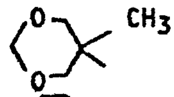 | Imidazol-1-yl | zähes Oel |
| II-10 | 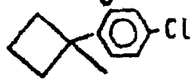 | 1,2,4-Triazol-1-yl | 92 |
| II-11 | 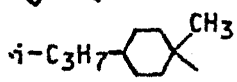 | Imidazol-1-yl | 53 |
| II-12 | $CH_3ON{=}CH{-}C(CH_3)_2{-}$ | 1,2,4-Triazol-1-yl | 1,4944 |
| II-13 | 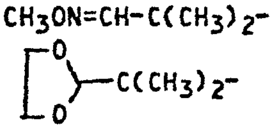 | 1,2,4-Triazol-1-yl | 198-200(x 1/2 NDS)[3)] |
| II-14 | 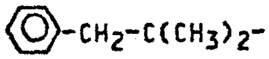 | 1,2,4-Triazol-1-yl | 79 |
| II-15 | 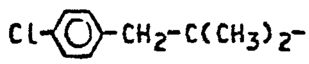 | 1,2,4-Triazol-1-yl | 127 |
| II-16 | 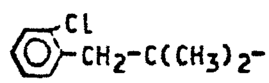 | 1,2,4-Triazol-1-yl | 66 |
| II-17 | 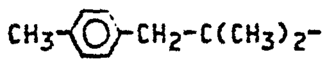 | 1,2,4-Triazol-1-yl | 100-02 |
| II-18 | 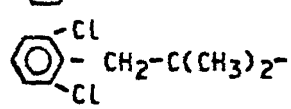 | 1,2,4-Triazol-1-yl | 1,5670 |
| II-19 | 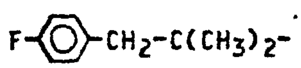 | 1,2,4-Triazol-1-yl | 93 |
| II-20 | 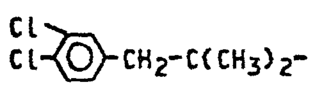 | 1,2,4-Triazol-1-yl | 114 |
| II-21 | 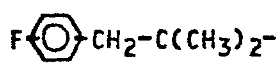 | Imidazol-1-yl | 54 |
| II-22 | 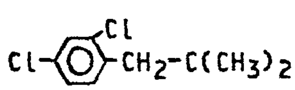 | 1,2,4-Triazol-1-yl | 85 |
| II-23 | 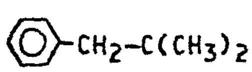 | Imidazol-1-yl | 65 |
| II-24 | 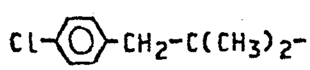 | Imidazol-1-yl | zähes Oel |
| II-25 | 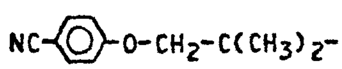 | Imidazol-1-yl | zähes Oel |

**Tabelle B** (Fortsetzung)

| Bsp. Nr. | R | Az | Fp(°C) bzw. Kp(°C)/mbar bzw. $n_D^{20}$ bzw. $R_f$ |
|---|---|---|---|
| II-26 | 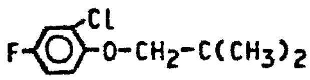 | Imidazol-1-yl | 74 |
| II-27 | 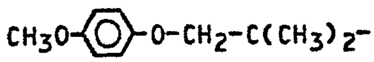 | Imidazol-1-yl | 1,5392 |
| II-28 | 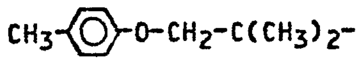 | Imidazol-1-yl | 1,5524 |
| II-29 | $C_2H_5-O-CH_2-C(CH_3)_2-$ | Imidazol-1-yl | zähes Oel |
| II-30 | $CH_3-O-CH_2-C(CH_3)_2-$ | 1,2,4-Triazol-1-yl | 1,4816 |
| II-31 | $C_2H_5-O-CH_2-C(CH_3)_2-$ | 1,2,4-Triazol-1-yl | 68-72(x HCl) |
| II-32 | $CH_3-O-CH_2-C(CH_3)_2-$ | Imidazol-1-yl | zähes Oel |
| II-33 | 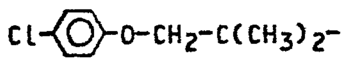 | Imidazol-1-yl | zähes Oel |
| II-34 | 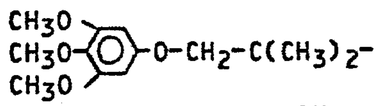 | Imidazol-1-yl | 137-38 |
| II-35 | 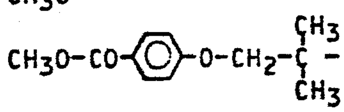 | Imidazol-1-yl | 1,5583 |
| II-36 | 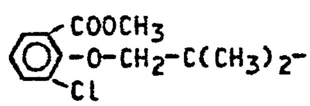 | Imidazol-1-yl | 1,5499 |
| II-37 | 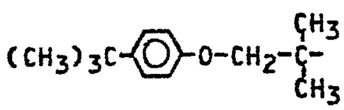 | Imidazol-1-yl | 1,5371 |
| II-38 | 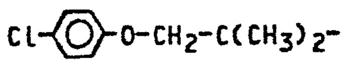 | 1,2,4-Triazol-1-yl | 90-92 |
| II-39 | 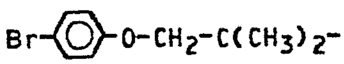 | 1,2,4-Triazol-1-yl | zähes Oel |
| II-40 | 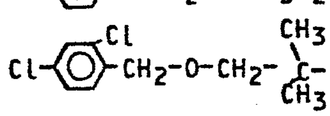 | 1,2,4-Triazol-1-yl | 1,5428 |
| II-41 | 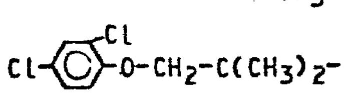 | 1,2,4-Triazol-1-yl | 1,5392 |
| II-42 | 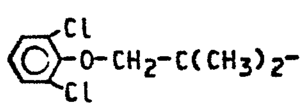 | 1,2,4-Triazol-1-yl | 1,5428 |
| II-43 | 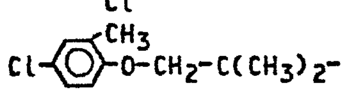 | 1,2,4-Triazol-1-yl | 93-95 |

**Tabelle B (Fortsetzung)**

| Bsp. Nr. | R | Az | Fp(°C) bzw. Kp(°C)/mbar $n_D^{20}$ bzw. $R_f$ |
|---|---|---|---|
| II-44 | 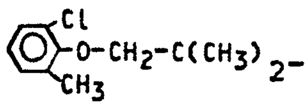 | 1,2,4-Triazol-1-yl | 170-75(xHCl) |
| II-45 | 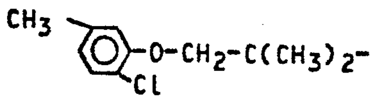 | 1,2,4-Triazol-1-yl | 146-47(xHCl) |
| II-46 | 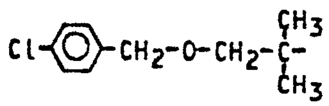 | 1,2,4-Triazol-1-yl | 1,5298 |
| II-47 | 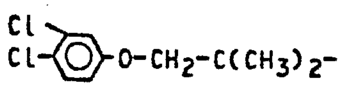 | 1,2,4-Triazol-1-yl | 108-110 |
| II-48 | 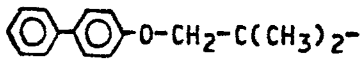 | 1,2,4-Triazol-1-yl | δ [ppm] DMSO [1] 1,35(s); 4,1(s); 5,62(s) (xHCl) |
| II-49 | 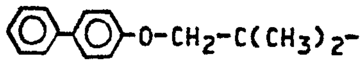 | Imidazol-1-yl | δ [ppm] DMSO [1] 1,35(s); 4,1(s); 5,65(s) (xHCl) |
| II-50 | 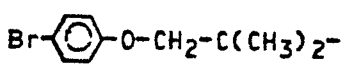 | Imidazol-1-yl | 165-68(xHCl) |
| II-51 | $CH_3-O-CH_2-C(CH_3)_2-$ | 1,2,4-Triazol-4-yl | 112 |
| II-52 | 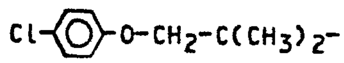 | 1,2,4-Triazol-4-yl | 124 |
| II-53 | 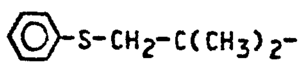 | 1,2,4-Triazol-1-yl | 1,5752 |
| II-54 | 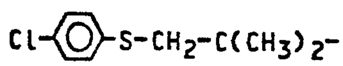 | 1,2,4-Triazol-1-yl | 1,5703 |
| II-55 | 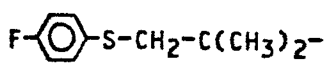 | 1,2,4-Triazol-1-yl | zähes Oel |
| II-56 | 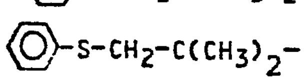 | 1,2,4-Triazol-4-yl | 110 |
| II-57 | 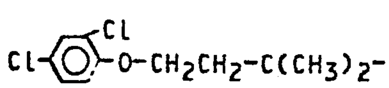 | 1,2,4-Triazol-1-yl | 62 |
| II-58 | 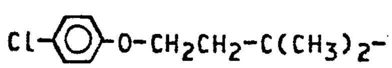 | 1,2,4-Triazol-1-yl | 1,5457 |
| II-59 | 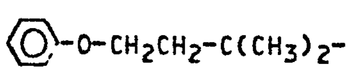 | 1,2,4-Triazol-1-yl | Harz |
| II-60 | 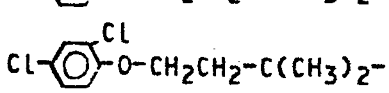 | Imidazol-1-yl | 46-48 |
| II-61 | 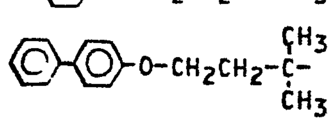 | Imidazol-1-yl | 132-34 |

0139227

Tabelle B (Fortsetzung)

| Bsp. Nr. | R | Az | Fp(°C) bzw. Kp(°C)/mbar bzw. $n_D^{20}$ bzw. $R_f$ |
|---|---|---|---|
| II-62 | 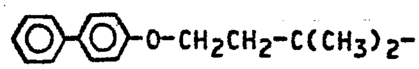 | 1,2,4-Triazol-1-yl | 125-27 |
| II-63 | 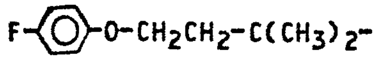 | Imidazol-1-yl | 1,5290 |
| II-64 | 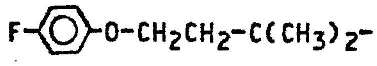 | 1,2,4-Triazol-1-yl | 1,7238 |
| II-65 | 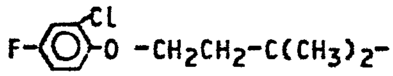 | Imidazol-1-yl | 69-70 |
| II-66 | 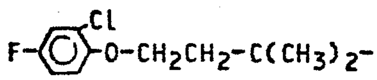 | 1,2,4-Triazol-1-yl | 54-56 |
| II-67 | 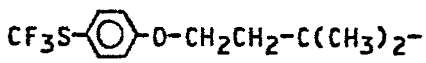 | Imidazol-1-yl | 1,5233 |
| II-68 | 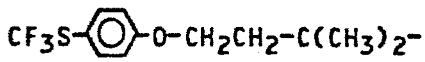 | 1,2,4-Triazol-1-yl | 72-77 |
| II-69 | 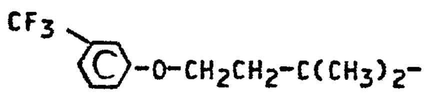 | Imidazol-1-yl | 1,5025 |
| II-70 | 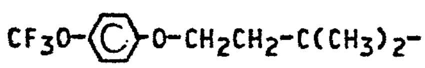 | Imidazol-1-yl | 1,4939 |
| II-71 | 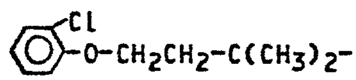 | Imidazol-1-yl | 78-79 |
| II-72 | 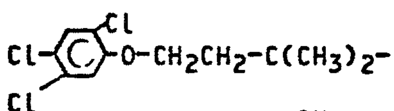 | Imidazol-1-yl | 127-32 |
| II-73 | 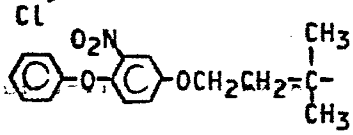 | Imidazol-1-yl | 82-83 |
| II-74 | 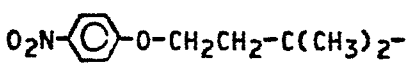 | 1,2,4-Triazol-1-yl | 151-53 |
| II-75 | 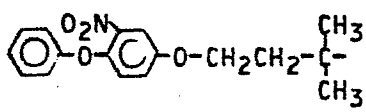 | 1,2,4-Triazol-1-yl | 101-02 |
| II-76 | 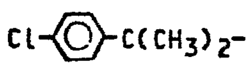 | Imidazol-1-yl | 1,5636 |
| II-77 | $n\text{-}C_3H_7\text{-}C(CH_3)_2\text{-}$ | 1,2,4-Triazol-1-yl | zähes Oel |
| II-78 | $CH_2\text{=}CH\text{-}C(CH_3)_2\text{-}$ | Imidazol-1-yl | 107/10/0,05 |
| II-79 | $CH_2\text{=}CH\text{-}C(CH_3)_2\text{-}$ | 1,2,4-Triazol-1-yl | 93-98/0,04 |

**Tabelle B (Fortsetzung)**

| Bsp. Nr. | R | Az | Fp(°C) bzw. Kp(°C)/mbar bzw. $n_D^{20}$ bzw. $R_f$ |
|---|---|---|---|
| II-80 | $i-C_3H_7-C(CH_3)_2-$ | 1,2,4-Triazol-1-yl | 110/0,05 |
| II-81 | $(CH_3)_3C-CH_2-C(CH_3)_2-$ | 1,2,4-Triazol-1-yl | 1,4851 |
| II-82 | $(CH_3)_3C-CH_2-C(CH_3)_2-$ | Imidazol-1-yl | 54-56 |
| II-83 | $i-C_3H_7-C(CH_3)_2-$ | 1,2,4-Triazol-4-yl | 155-56 |
| II-84 | $CH_3-C(CH_3)_2-$ | 1,2,4-Triazol-1-yl | 65-67 |
| II-85 | $ClCH_2-C(CH_3)_2-$ | Imidazol-1-yl | zähes Oel |
| II-86 | $CH_3-C(CH_3)_2-$ | Imidazol-1-yl | 135 |
| II-87 | $FCH_2-C(CH_3)_2-$ | Imidazol-1-yl | δ [ppm]$CDCl_3$ 1)<br>1,2(s); 4,0(s);<br>4,75(s);4,95(s) |
| II-88 | $CF_3S-C(CH_3)_2-$ | 1,2,4-Triazol-1-yl | δ [ppm]DMSO 1)<br>1,75(s);5,8(s) |
| II-89 | $NC-CH_2CH_2-C(CH_3)_2-$ | 1,2,4-Triazol-1-yl | 155-58(xHCl) |
| II-90 | 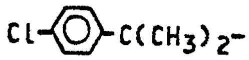 | 1,2,4-Triazol-1-yl | 60 |
| II-91 | 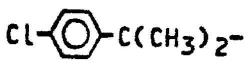 | 1,2,4-Triazol-4-yl | 168 |
| II-92 | $C_2H_5O-CO-C(CH_3)_2-$ | 1,2,4-Triazol-1-yl | 1,4769 |
| II-93 | 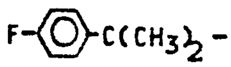 | 1,2,4-Triazol-1-yl | 1,5268 |
| II-94 | 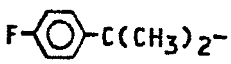 | 1,2,4-Triazol-4-yl | 184 |
| II-95 | 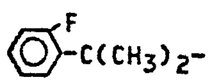 | 1,2,4-Triazol-4-yl | 134 |
| II-96 | 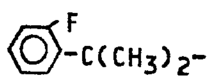 | 1,2,4-Triazol-1-yl | 85 |
| II-97 | $CH_3-C(CH_3)_2-$ | 1,2,4-Triazol-4-yl | 187 |
| II-98 | $CH_3-C(CH_3)_2-$ | Pyrazol-1-yl | 57-60 |
| II-99 | 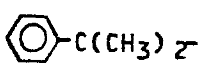 | 1,2,4-Triazol-1-yl | zähes Oel |
| II-100 | $ClCH_2-C(CH_3)_2-$ | 1,2,4-Triazol-1-yl | 1,505 |
| II-101 | $C_2H_5O-CO-C(CH_3)_2-$ | Imidazol-1-yl | zähes Oel |

**Tabelle B** (Fortsetzung)

| Bsp. Nr. | R | Az | Fp(°C) bzw. Kp(°C)/mbar bzw. $n_D^{20}$ bzw. $R_f$ |
|---|---|---|---|
| II-102 | $C_2H_5O-CO-C(CH_3)_2-$ | Pyrazol-1-yl | zähes Oel |
| II-103 | $BrCH_2-C(CH_3)_2-$ | 1,2,4-Triazol-1-yl | 150-54/0,2 |
| II-104 | $FCH_2-C(CH_3)_2-$ | 1,2,4-Triazol-1-yl | Kristallbrei |
| II-105 | $NC-C(CH_3)_2-$ | 1,2,4-Triazol-1-yl | 1,5012 |
| II-106 | $C(CH_3)(CH_2Cl)_2-$ | Imidazol-1-yl | $R_f$=0,38 $CHCl_3$:MeOH=9:1 [2)] |
| II-107 | $C(CH_3)(CH_2F)_2-$ | 1,2,4-Triazol-1-yl | 1,519 |
| II-108 | $C(CH_3)(CH_2F)_2-$ | 1,2,4-Triazol-1-yl | zähes Oel |
| II-109 | Cl 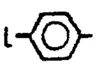 S-$CH_2$-$C(CH_3)_2$- | Imidazol-1-yl | 50 |

[1)] Zu den NMR-Daten der Beispiele II-48, II-49, II-87 und II-88:

DMSO = Dimethylsulfoxid (als Lösungsmittel)
s = Sigulett

[2)] MeOH = $CH_3OH$

[3)] NDS = 1.5-Naphthalin-disulfonsäure

Patentansprüche

1. Herbizide Mittel, gekennzeichnet durch einen Gehalt an einer Wirkstoffkombination, bestehend aus

(a) einem Photosynthesehemmer-Wirkstoff (Herbizid) und

(b) einem Azolylmethylketon der allgemeinen Formel (II) (Synergist),

$$R - CO - CH_2 - Az \qquad (II)$$

in welcher

R für einen organischen Rest steht und
Az für einen Azolrest steht,

oder einem von deren Säureadditions-Salzen.

2. Herbizide Mittel gemäß Anspruch 1, dadurch gekennzeichnet, daß sie als Photosynthesehemmer-Wirkstoffe (Herbizide) Verbindungen aus den nachfolgend genannten Wirkstoffgruppen der Formeln (I-A) bis (I-J) enthalten:

(A) Triazinon-Derivate der Formel

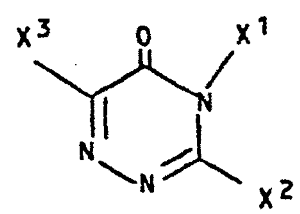

(I-A)

in welcher

$X^1$ für Amino, gegebenenfalls substituiertes Alkylidenamino oder Alkyl mit 1 bis 2 Kohlenstoffatomen steht;

$X^2$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^3$ für gegebenenfalls durch Halogen substituiertes tert.-Butyl oder gegebenenfalls substituiertes Phenyl steht;

(B) Triazindion-Derivate der Formel

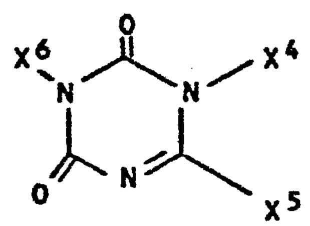

(I-B)

in welcher

$X^4$ für Amino, gegebenenfalls substituiertes Alkylidenamino oder Alkyl mit 1 bis 2 Kohlenstoffatomen steht;

$X^5$ für Alkylthio mit 1 bis 2 Kohlenstoffatomen, Alkyl- und Dialkylamino mit jeweils 1 bis 2 Kohlenstoffatomen in jedem Alkylteil oder Alkyl mit 1 bis 4 Kohlenstoffatomen steht; und

$X^6$ für Alkyl mit 1 bis 6 Kohlenstoffatomen oder gegebenenfalls substituiertes Phenyl steht;

(C) Triazin-Derivate der Formel

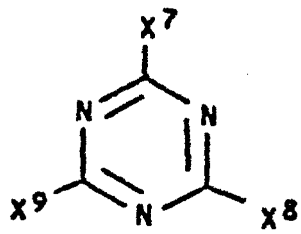

(I-C)

in welcher

$X^7$ für Chlor, Alkoxy oder Alkylthio mit jeweils 1 bis 2 Kohlenstoffatomen steht;

$X^8$ für Alkylamino mit 1 bis 4 Kohlenstoffatomen im Alkylteil steht; und

$X^9$ für gegebenenfalls durch Cyano substituiertes Alkyl mit 1 bis 4 Kohlenstoffatomen steht;

(D) Harnstoff-Derivate der Formel

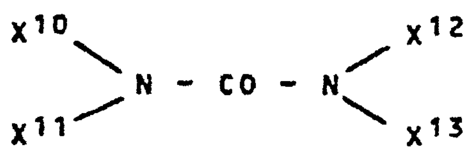

(I-D)

in welcher

$X^{10}$ für gegebenenfalls substituiertes Phenyl, Benz-thiazolyl oder gegebenenfalls substituiertes Thiadiazolyl steht;

$X^{11}$ für Wasserstoff oder Methyl steht;

$X^{12}$ für Methyl steht; und

$X^{13}$ für Wasserstoff, Alkyl mit 1 bis 4 Kohlenstoffatomen, Alkoxy mit 1 bis 2 Kohlenstoffatomen oder Alkinyl mit 2 bis 4 Kohlenstoffatomen steht;

(E) Carboxanilid-Derivate der Formel

$$X^{14} - CO - NH - X^{15} \qquad \text{(I-E)}$$

in welcher

$X^{14}$ für Alkyl mit bis zu 6 Kohlenstoffatomen, Alkoxy mit 1 bis 4 Kohlenstoffatomen, Alkenyl mit 2 bis 4 Kohlenstoffatomen oder Cycloalkyl mit 3 bis 6 Kohlenstoffatomen steht; und

$X^{15}$ für gegebenenfalls substituiertes Phenyl steht;

(F) Uracil-Derivate der Formel

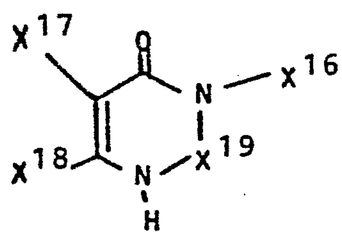

(I-F)

in welcher

$X^{16}$ für Alkyl mit 1 bis 6 Kohlenstoffatomen oder Cycloalkyl mit 5 bis 7 Kohlenstoffatomen steht;

$X^{17}$ für Halogen steht;

$X^{18}$ für Alkyl mit 1 bis 2 Kohlenstoffatomen steht, oder

$X^{17}$ und $X^{18}$ gemeinsam für eine gegebenenfalls substituierte Alkylenkette oder einen gegebenenfalls substituierten anellierten Benzolring stehen; und

$X^{19}$ für die -CO- oder $-SO_2$-Gruppe steht;

(G) Biscarbamat-Derivate der Formel

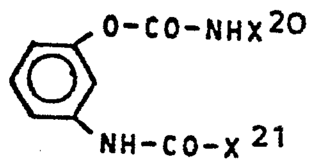

(I-G)

in welcher

$X^{20}$ für Alkyl mit 1 bis 4 Kohlenstoffatomen oder gegebenenfalls substituiertes Phenyl steht; und

$X^{21}$ für Alkoxy mit 1 bis 4 Kohlenstoffatomen oder Dialkylamino mit 1 bis 2 Kohlenstoffatomen in jedem Alkylteil steht;

(H) Pyridazinon-Derivate der Formel

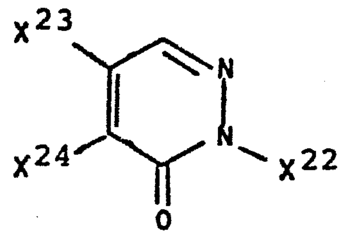

(I-H)

in welcher

$X^{22}$ für gegebenenfalls substituiertes Phenyl steht;

$X^{23}$ für Amino, Alkylamino oder Dialkylamino mit jeweils 1 oder 2 Kohlenstoffatomen in jedem Alkylteil steht; und

$X^{24}$ für Halogen steht;

(J) Hydroxybenzonitril-Derivate der Formel

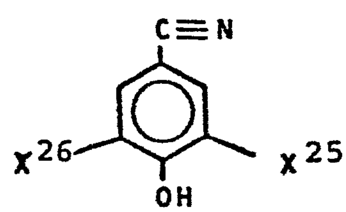

(I-J)

in welcher

$X^{25}$ für Halogen steht; und

$X^{26}$ für Halogen steht.

3. Herbizide Mittel gemäß Anspruch 1, dadurch gekennzeichnet, daß sie als Photosynthesehemmer-Wirkstoff (Herbizid) 4-Amino-6-tert.-butyl-3-methylthio-1,2,4-triazin-5-on der Formel (I-A-1) (Metribuzin)

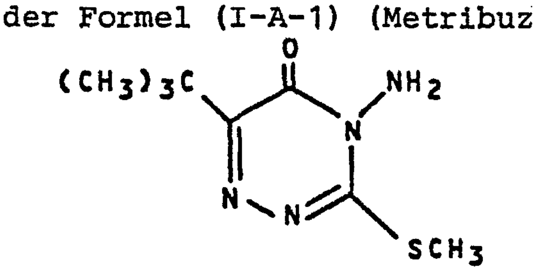

enthalten.

4. Herbizide Mittel gemäß Anspruch 1, dadurch gekennzeichnet, daß in der Wirkstoffkombination das Gewichtsverhältnis von (1) Photosynthesehemmer-Wirkstoff (Herbizid) zu (2) dem Azolylmethylketon der Formel (II) (Synergist) zwischen 1 : 0,25 und 1 : 100 liegt.

5. Herbizide Mittel gemäß Anspruch 4, dadurch gekennzeichnet, daß das angegebene Gewichtsverhältnis zwischen 1 : 5 und 1 : 50 liegt.

6. Herbizide Mittel gemäß Anspruch 5, dadurch gekennzeichnet, daß das angegebene Gewichtsverhältnis zwischen 1 : 10 und 1 : 20 liegt.

7. Verwendung von Wirkstoffkombinationen gemäß Ansprüchen 1 bis 6 zur Bekämpfung von Unkraut.

8. Verfahren zur Herstellung von herbiziden Mitteln, dadurch gekennzeichnet, daß man eine Wirkstoffkombination gemäß Ansprüchen 1 bis 6 mit Streckmitteln und/oder oberflächenaktiven Mitteln vermischt.

9. Verwendung von Azolylmethylketonen der Formel (II) gemäß Anspruch 1 als Synergisten in Kombination mit Photosynthesehemmer-Herbiziden.

10. Verwendung von Azolylmethylketonen der Formel (II) gemäß Anspruch 1 als Synergisten in Kombination mit dem herbiziden Wirkstoff 4-Amino-6-tert.-butyl-3-methylthio-1,2,4-triazin-5-on (Metribuzin) der Formel (I-A-1) gemäß Anspruch 3.